# EUROPÄISCHE PATENTANMELDUNG

(11) **EP 2 508 138 A1**
(43) Veröffentlichungstag der Anmeldung: **10.10.2012**
(21) Anmeldenummer: 12162994.3
(22) Anmeldetag: 03.04.2012
(51) Int. Cl.: A61B 17/04

(54) **Instrumentarium zum knotenfreien Fixieren von Gewebe an einem Knochen**

(30) Priorität: 04.04.2011 DE 102011016659
(71) Anmelder: Karl Storz GmbH & Co. KG, 78532 Tuttlingen (DE)
(72) Erfinder: Frey, Sebastian, 68753 Waghäusel (DE)
(74) Vertreter: Witte, Weller & Partner

(57) **Zusammenfassung**

Ein Instrumentarium (10) dient zum knotenfreien Fixieren von Gewebe an einem Knochen. Es weist ein bolzenförmiges, eine Längsachse aufweisendes Ankerelement (12) auf, das mit einem Faden verbunden ist, der einen endseitigen Schlaufenabschnitt (32) aufweist. Es wird vorgeschlagen, den Schlaufenabschnitt (32) zu einem Ringkörper (33) zu formen, dessen lichter Durchmesser so gewählt ist, dass das Ankerelement (12) durch den Ringkörper (33) hindurch bewegbar ist. Die Länge des Abschnittes (38, 38') des Fadens zwischen Ankerelement (12) und Ringkörper (33) ist derart aufwählbar, dass nach dem Durchbewegen des Ankerelementes (12) durch den Ringkörper (33) eine Schlaufe (40) ausbildbar ist, die umfänglich um ein zu fixierendes strangförmiges Gewebestück (72) liegt.

## Beschreibung

Die Erfindung betrifft ein Instrumentarium zum knotenfreien Fixieren von Gewebe an einem Knochen, mit einem bolzenförmigen, eine Längsachse aufweisenden Ankerelement, das mit einem Faden verbunden ist, der einen endseitigen Schlaufenabschnitt aufweist.

Derartige Instrumentarien oder Ankersysteme dienen dazu, ein von einem Knochen abgelöstes Gewebe wieder an diesem zu fixieren.

Die Notwendigkeit besteht bei Verletzungen an der Schulter, an der Hüfte oder dergleichen. Dort existieren um die Gelenke manschettenartige oder kranzartige Gewebebereiche, die am Knochen angewachsen sind. Bei Verletzungen, hauptsächlich bei Sportverletzungen, werden meist strangförmige Abschnitte des Gewebes vom Knochen einseitig abgelöst.

Eine typische Verletzung am Schultergelenk entsteht beispielsweise bei Handballspielern, die bei einem Torwurf abrupt blockiert werden.

Das Grundsprinzip der Fixierung besteht nun darin, einen Anker vorzusehen, der mit einem Faden verbunden ist. Der Faden dient dazu, um den Anker mit dem losgelösten Sehnenendstück zu verbinden.

Das Ankerelement selbst wird in den Knochen eingebracht, sei es in eine vorgebohrte Öffnung eingesetzt oder direkt in den Knochen eingeschlagen. Dadurch wird die abgelöste Sehne, die über den Faden mit dem Anker verbunden ist, wieder an dem Knochen fixiert.

In den Anfängen dieser Technologie wurde der Anker mit von diesem abstehenden losen Fadenenden in den Knochen eingeschlagen und die Fadenenden wurden mit dem abgelösten Gewebestück verbunden und anschließend verknotet.

Dabei wurde festgestellt, dass diese Knoten unerwünschte Erhebungen unter der Haut bilden und den späteren Heilprozess stören.

Daher wurde diese Technologie dahingehend weiterentwickelt, dass sogenannte knotenfreie Ankersysteme entwickelt wurden. Dabei steht von dem Anker ein Faden in Form einer Schlaufe vor, die mit dem zu fixierenden Sehnenabschnitt verbunden wird.

Die Spannung bzw. das möglichst nahe Fixieren des abgerissenen Gewebes am Knochen wird also nicht dadurch bewerkstelligt, dass ein Faden entsprechend gespannt und dann verknotet wird, sondern dass die Schlaufe durch entsprechende Konstruktion des Ankerelementes gespannt werden kann.

Derartige Vorrichtungen bzw. derartige Instrumentarien sind beispielsweise aus der DE 10 2006 010 116 A1, der EP 1 199 036 B1 oder der WO 2005/102190 A2 bekannt.

Es ist Aufgabe der Erfindung, ein solches Instrumentarium zum knotenfreien Fixieren dahingehend weiterzuentwickeln, dass das Fixieren sicher, gezielt und einfach durchführbar ist.

Erfindungsgemäß wird die Aufgabe dadurch gelöst, dass der Schlaufenabschnitt zu einem Ringkörper geformt ist, dessen lichter Durchmesser so gewählt ist, dass das Ankerelement durch den Ringkörper hindurch bewegbar ist, und dass eine Länge des Abschnittes des Fadens zwischen Ankerelement und dem Ringkörper derart auswählbar ist, dass, nach Hindurchbewegen des Ankerelements durch den Ringkörper, eine Schlaufe ausbildbar ist, die umfänglich eng um ein zu fixierendes strangförmiges Gewebestück liegt.

Diese Maßnahmen haben nun zahlreiche Vorteile.

Die Grundkonstruktion ist so, dass das Ankerelement über einen Abschnitt des Fadens, der eine bestimmte Länge aufweist, mit dem Schlaufenabschnitt verbunden ist, der zu einem Ringkörper geformt ist. Durch Auswahl des lichten Innendurchmessers des Ringkörpers derart, dass das Ankerelement durch diesen Ringkörper hindurch geschoben werden kann, schafft, nach diesem Hindurchschieben, eine neue Schlaufe.

Diese nun neu gebildete Schlaufe kann dazu herangezogen werden, um ein strangförmiges Gewebestück gelegt zu werden. Durch Ziehen an dem durch den Ringkörper bereits hindurchbewegten Ankerelement wird diese Schlaufe immer enger, d.h. legt sich immer enger um das zu fixierende strangförmige Gewebestück an. Das wird in der Praxis durch die Einschlagtiefe des Ankerelementes in den Knochen geregelt. Da der Operateur weiß, wie dick etwa dieses strangförmige Gewebestück ist, weiß er auch, wie lang er die Länge des Abschnittes des Fadens zwischen dem Ringkörper und dem Ankerelement auswählen muss, um eine Schlaufe auszubilden, die das strangförmige Gewebestück eng umgibt und entsprechend unter Spannung an dem Ringkörper bzw. in Richtung auf das Ankerelement zieht.

Es ist möglich, diese Länge vor Ort individuell anzupassen, oder schon herstellerseitig aufgrund von Erfahrungswerten bereitzustellen. Es ist bekannt, dass beispielsweise die Höhe einer eine menschliche Schulter umrundende Manschette ein gewisses Maß aufweist, d.h. es ist auch dann bekannt, welchen Durchmesser ein Strang an von dieser Manschette teilweise abgerissenem Gewebe aufweist.

Bei der Handhabung wird der Zusammenbau aus Ankerelement, von diesem abstehenden Abschnitt des Fadens und dem Ringkörper seitlich um das strangförmige Gewebestück gelegt. Dabei kann der Ringkörper bereits an der Stelle an die Knochenfläche angelegt werden, an der das Ankerelement eingetrieben werden soll. Danach wird das Ankerelement mittig in dem Ringkörper angesetzt, wodurch sich dann schon der Abschnitt des Fadens zwischen Ankerelement und Ringkörper um das strangförmige Gewebestück gelegt hat. Durch Durchtreiben des Ankerelements durch den Ringkörper und Eintreiben in den Knochen wird dann diese Schlaufe um das strangförmige Gewebestück straff gelegt und zieht es an den Knochen heran. Die Einschlagtiefe des Ankerelementes in den Knochen bestimmt somit das Maß der Spannung der Schlaufe, um das strangförmige Gewebestück zu fixieren.

Der Operateur braucht keine aufwändigen Montagemaßnahmen vorzunehmen, wie das beispielsweise bei Ankerstücken der Fall ist, die aus mehrteiligen Elementen bestehen. Der Fixiervorgang ist einfach durchzuführen, d.h. der Zusammenbau aus Ankerelement, Faden und Ringkörper ist nur um das strangförmige Gewebestück zu legen und dann das Ankerelement durch den Ringkörper hindurch in den Knochen einzutreiben.

Der Ringkörper, der aus dem Faden selbst gebildet ist, kann, bei entsprechendem Material des Fadens, als Ringkörper vorgeformt sein, sofern das Fadenmaterial eine entsprechend ausreichende Formstabilität aufweist. Dieser Ringkörper kann auch dadurch ausgebildet sein, dass der Abschnitt zwischen Ankerelement und dem auszubildenden Ringkörper durch zwei Fadenstücke eines einzigen Fadens gebildet wird, der insgesamt gesehen als Schlaufe ausgebildet ist. Wenn, wie gesagt, das Material es erlaubt, den Ringkörper entsprechend formstabil auszubilden, ist dann der Längenabschnitt des Fadens zwischen dem Ringkörper und dem Ankerelement als Doppelstrang ausgebildet. Es können auch entsprechende Hilfsmaßnahmen ergriffen werden, um den Ringkörper als solchen aufrecht zu erhalten, indem beispielsweise an der Stelle des Ringkörpers, von der dieser Doppelstrang weg führt, ein kleiner Clip oder ein Befestigungspunkt, beispielsweise durch eine Klebestelle oder dergleichen, ausgebildet ist.

Nimmt man in Kauf, dass nach Krafteinwirkung beim Durchschlagen des Ankerelements durch den Ringkörper dieser sich öffnet und dieser Fadenabschnitt ein Bestandteil der Schlaufe bildet, die sich insgesamt um das strangförmige Gewebestück legt, so muss man diesen Längenabschnitt entsprechend mit hinzurechnen, da man ansonsten das Ankerelement unnötig tief in den Knochen einschlagen müsste.

Entscheidend ist, dass dieser Ringkörper zunächst vorhanden ist, um der Handhabungsperson eine klare Orientierung zu geben, wo er das Ankerelement hindurchführen soll. In dem Fall, in dem sich danach die Ringgeometrie auflöst, muss dann die halbe Länge des Umfanges dieses Ringkörpers zu der Länge des Abschnittes des Fadens zwischen dem ursprünglichen Ringkörper und dem Ankerelement hinzugerechnet werden, um letztendlich eine Schlaufenlänge zu erzielen, die gerade ausreichend ist, um sie umfänglich eng um das strangförmige Gewebestück anzulegen und durch entsprechendes Einschlagen des Ankerelements in dem Knochen die ausreichende Zugspannung aufzubauen. Die Länge des Fadens, also die Länge des Fadenabschnittes plus der halbe Umfang des Ringkörpers sind so zu wählen, dass das Gewebestück fest umschlungen ist.

In einer weiteren Ausgestaltung der Erfindung ist der Schlaufenabschnitt um ein Ringelement geführt.

Diese Maßnahme hat den Vorteil, dass die Schlaufengeometrie durch dieses zusätzliche Ringelement bestimmt wird, so dass auch bei sehr biegeschlaffen Fadenmaterialien die Ringgeometrie aufrecht erhalten bleibt. Dieses Ringelement verbleibt auch nach dem Durchtreiben des Ankerelements durch das Ringelement hindurch im Körper.

Dieses Ringelement stellt ein gut handhabbares Element dar, d.h. der Operateur kann es beispielsweise von Hand ergreifen, nachdem er den Zusammenbau um das strangförmige Gewebestück gelegt hat, und kann es an einer ganz bestimmten Stelle an den Knochen anlegen, die gerade günstig für das Eintreiben des Ankerstückes ist.

In einer weiteren Ausgestaltung der Erfindung ist das Ringelement als Ring ausgebildet, an dessen umfänglicher Außenfläche eine Aussparung vorhanden ist, in die der Schlaufenabschnitt einlegbar ist.

Diese Maßnahme hat den Vorteil, dass bereits herstellerseitig der ursprüngliche Schlaufenabschnitt in das Ringelement bzw. in die äußere Aussparung eingelegt werden kann und somit dann der entsprechende Ringkörper aus dem Fadenmaterial ausgebildet wird.

In einer weiteren Ausgestaltung der Erfindung ist die Aussparung derart ausgebildet, dass der Fadenabschnitt abrutschsicher anliegt.

Diese Maßnahme hat den Vorteil, dass beispielsweise herstellerseitig der Faden unter einem gewissen Anpressdruck in die Aussparung eingedrückt wird, so dass dann dieser Zusammenbau bei der Handhabung fest erhalten bleibt und sich der Ring nicht vom Faden löst bzw. umgekehrt.

Diese Handhabung ist zunächst einmal die Verpackung nach der Herstellung, das Öffnen der Verpackung an der Operationsstelle, das Bereitlegen für den Operateur und dann auch die Handhabung durch den Operateur beim Anlegen an das Gewebestück. Bei all diesen Manipulationen bleibt der Fadenabschnitt fest in dem Ring aufgenommen.

Dies erleichtert erheblich die Handhabung der Vorrichtung.

In einer weiteren Ausgestaltung der Erfindung weist das Ankerelement eine proximale Anbringstelle auf, an der der Faden anbringbar ist.

Diese Maßnahme hat den Vorteil, dass durch diese Anbringstelle die Länge des Fadenabschnittes, insbesondere die Länge des Abschnittes zwischen dem Ankerelement und dem Ringkörper, anpassbar ist. So kann mit ein und derselben körperlichen Ausgestaltung von Ankerelement und Ring ein Zusammenbau vorbereitet werden, der beispielsweise geeignet ist, um einen relativ dicken Strang an Gewebe gelegt zu werden oder, bei entsprechend kürzerer Ausbildung dieses Abschnittes zwischen Ankerelement und Ringkörper, bei an sich gleicher Konstruktion geeignet ist, um einen wesentlich dünneren Strang gelegt zu werden.

Dies eröffnet auch die Möglichkeit, direkt vor Ort zunächst einmal die Dicke des zu befestigenden Gewebestückes bzw. Stranges zu ermitteln und dann vor Ort den Faden so an der proximalen Anbringstelle anzubringen, dass optimale Verhältnisse resultieren.

Optimale Verhältnisse bedeutet, dass die nach Durchschieben des Ankerelements durch das Ringelement ausgebildete Schleife eng um die Außenseite des zu fixierenden Gewebes anliegt, so dass dieses fest gehalten ist und zugleich die Spannung schon dann aufgebaut ist, wenn der Anker gerade vollständig in den Knochen eingeschlagen ist. Das bedeutet, dass das proximale Ende des Ankers auf Höhe der Knochenfläche zum Liegen kommt. Dies resultiert dann in einem möglichst atraumatischen Einbringen des Ankerelements in den Knochen, ohne dass hier unnötig tief in den Knochen eingedrungen werden muss. Das führt nicht nur zu einer wesentlich atraumatischeren Verankerung, sondern auch zu einer sehr hohen Flexibilität im Hinblick auf zu fixierende Gewebestücke.

In einer weiteren Ausgestaltung der Erfindung ist die Anbringstelle eine umfängliche Nut an der Außenseite des Ankerkörpers, in die das ankerseitige Ende des Fadens einlegbar ist.

Diese Maßnahme hat den Vorteil, dass dadurch eine relativ stabile formschlüssige Verbindung zwischen dem Faden und dem Ankerelement zu bewerkstelligen ist.

Dabei wird das dem Ankerelement zugewandte Ende des Fadens einmal um diese umfängliche Nut geschlungen und dann entsprechend befestigt. Diese Befestigung kann durchaus durch einen Knoten oder ein sonstiges Klemmstück bewerkstelligt werden kann, das schon herstellseitig zur Verfügung gestellt werden kann. Für den Fall, dass eine Verknotung stattfinden soll, ist dann am Ankerelement eine entsprechende Ausnehmung oder Aussparung vorgesehen, um diesen Knoten aufnehmen zu können, so dass dieser nicht seitlich absteht. Dennoch bleibt das Prinzip des knotenfreien Ankers auch dann aufrecht erhalten, denn darunter versteht man, dass beim eigentlichen Fixieren des Gewebes kein neuer Knoten bewerkstelligt werden muss. Dies ist hier der Fall, denn die Fixierung erfolgt durch die neu entstehende Schlaufe, nachdem das Ankerelement durch den Ringkörper hindurchgetrieben worden ist.

In einer weiteren Ausgestaltung der Erfindung ist am proximalen Ende des Ankerelements ein Eingriffsmerkmal für ein Eintreibwerkzeug vorhanden.

Diese Maßnahme hat den Vorteil, dass ein weiteres Bauelement des Instrumentariums, nämlich ein Eintreibwerkzeug, an das Ankerelement angesetzt werden kann, um dieses durch den Ringkörper hindurch in den Knochen einzutreiben.

In einer weiteren Ausgestaltung ist das Ankerelement als massiver zylindrischer Körper ausgebildet, dessen distales Ende als kegelartige Spitze ausgebildet ist.

Diese Maßnahme hat den Vorteil, dass das Ankerelement beispielsweise über die Spitze direkt in den Knochen eingeschlagen werden kann, ohne dass vorher eine Bohrung bewerkstelligt werden muss.

Dies erhöht auch die Flexibilität, denn der Operateur kann sich vorab, nach Anlegen des Zusammenbaus an das zu fixierende Gewebestück, dann eine günstige Stelle am Knochen heraussuchen, an der er den Ringkörper anlegt und dann entsprechend die Spitze des Ankerelements ansetzt, um dieses einzuschlagen. Müsste vorher eine Bohrung bewerkstelligt werden, wäre er dann an die Lage und Orientierung dieser Bohrung gebunden. Somit erhöht auch dies die Flexibilität des Einsatzes eines solchen Instrumentariums.

In einer weiteren Ausgestaltung der Erfindung ist das Eingriffsmerkmal als eine sich in Längsrichtung erstreckende Bohrung im Körper des Ankerelements ausgebildet.

Diese an sich bekannte Maßnahme hat den Vorteil, dass der Eingriff zwischen dem Eintreibwerkzeug und dem Ankerelement ganz einfach durch diese Bohrung erfolgen kann, indem nämlich in diese Bohrung ein entsprechender Vorsprung des Eintreibwerkzeuges eingeschoben wird.

In einer weiteren Ausgestaltung der Erfindung ist die Bohrung durch den Körper vollständig hindurchgehend und die kegelartige Spitze ist als Kegelstumpf ausgebildet, und ein in die Bohrung eingeführtes Eintreibwerkzeug weist eine Spitze auf, die den Kegelstumpf zu einem spitzen Kegel ergänzt.

Diese Maßnahme hat den Vorteil, dass die über den Kegelstumpf vorstehende Spitze des Eintreibwerkzeuges entsprechend stabil und massiv bzw. aus Metall ausgebildet werden kann, um den Zusammenbau einfach und zielgerichtet in den Knochen eintreiben zu können.

Nach Abziehen des Eintreibwerkzeuges verbleibt das Ankerelement im Körper, wobei dann nur der stumpfe Kegelstumpf das distale vordere Ende des Ankerelements darstellt. Dadurch kann das Ankerelement selbst aus anderen auch weniger schlagstabilen Materialien hergestellt werden. Das erlaubt es auch insgesamt, das Ankerelement als relativ kleines schwaches Bauteil auszubilden, so dass beispielsweise an Knochenbereichen, die nicht so großflächig wie die Schulter oder die Hüfte ist, auch solche Fixiervorgänge durchgeführt werden können.

In einer weiteren Ausgestaltung der Erfindung ist eine Haltevorrichtung vorgesehen, an der ein mit dem Ankerelement verbundenes Eintreibwerkzeug verbunden ist, und die distalseitig eine Klammer aufweist, in die das Ringelement samt Fadenabschnitt seitlich einclipsbar ist.

Diese Maßnahme hat den Vorteil, dass die Haltevorrichtung das Eintreibwerkzeug in einer bestimmten Ausrichtung hält, wobei dann an das Eintreibwerkzeug das Ankerelement anlegbar ist. Durch die distalseitige Klammer kann das Ringelement zugleich in die richtige Ausrichtung gebracht werden, so dass Ankerelement und Ringelement in der Orientierung zueinander stehen, bei der das Ankerelement nur noch durch eine lineare Vorschubbewegung durch das Ringelement hindurchgeschlagen werden muss. Durch Ausbildung einer Klammer, in der das Ringelement einclipsbar ist, ist dieser Zusammenbau einfach zu bewerkstelligen und selbstverständlich auch dann wieder zu lösen, nachdem das Ankerelement entweder schon teilweise oder vollständig durch das Ringelement hindurchgetrieben worden ist.

In einer weiteren Ausgestaltung der Erfindung weist die Haltevorrichtung eine seitlich zur Längsachse verlaufende Führung für den Fadenabschnitt zwischen Ringkörper und Ankerelement auf.

Diese Maßnahme hat den Vorteil, dass durch diese Führung dieser Fadenabschnitt zwischen Ringkörper und Ankerelement in einer bestimmten Orientierung liegt, was es dann dem Operateur erleichtert, diesen Fadenabschnitt seitlich an das zu fixierende Gewebe heranzubewegen und anzulegen.

In einer weiteren Ausgestaltung der Erfindung ist die Führung als seitlich ausbauchender hochstehender Bügel ausgebildet, an dessen dem Fadenabschnitt zugewandten Seite eine Ausnehmung zur Aufnahme des Fadenabschnitts vorgesehen ist.

Diese Maßnahme hat den Vorteil, dass in den ausbauchenden Bügel in dessen Ausnehmung dieser Fadenabschnitt gehalten und geführt aufgenommen werden kann.

Das bedeutet, dass beispielsweise dieser komplette Zusammenbau des Instrumentariums schon vor der Operation bewerkstelligt werden kann. Wenn vor dem operativen Eingriff individuell auf den Durchmesser des Gewebes angepasst werden soll, kann das eine Hilfsperson durchführen. Dem Operateur wird dann das vorbereitete Instrumentarium gereicht, er führt es seitlich an den zu fixierenden Gewebestrang an, legt die Klammer mit dem Ringelement auf den Knochen auf und braucht dann nur noch auf das Eintreibwerkzeug zu schlagen, um dies mit dem daran zu befestigenden Ankerelement zielgerichtet durch das Ringelement hindurch in den Knochen einzutreiben.

In einer weiteren Ausgestaltung der Erfindung ist das Eintreibwerkzeug höhenverschiebbar am proximalen Ende des Bogens angebracht.

Diese Maßnahme hat den Vorteil, dass eine weitere individuelle Anpassbarkeit möglich ist.

In einer weiteren Ausgestaltung der Erfindung ist die Klammer, die am proximalen Ende des Bogens angeordnet ist, und der Ringkörper des Fadenabschnittes in einer solchen Ausrichtung gehalten, dass der Ringkörper an einer Knochenaußenseite flächig anlegbar ist.

Diese Maßnahme hat den Vorteil, dass dadurch dem Operateur die Heranführung, Ausrichtung und das Anlegen des Instrumentariums an die Knochenfläche erheblich erleichtert ist.

In einer weiteren Ausgestaltung der Erfindung ist der Ringkörper in einer Ausrichtung in der Klammer gehalten, bei der die Längsachse des Ankerelements mittig senkrecht auf der Ringebene steht.

Diese Geometrie erlaubt es dem Operateur, die Klammer zielgerichtet anzusetzen und ihm auch schon visuell die Schlagrichtung bzw. Eintreibrichtung vorzugehen, in der das Ankerelement eingetrieben werden soll. Wenn er weiß, wie lang etwa dieses Ankerelement ist, weiß er dann in welcher Ausrichtung er das Instrumentarium ansetzen muss, um auf jeden Fall genügend Knochenmaterial zur Verfügung zu haben, in das das Ankerelement eingetrieben werden kann.

Auch das erhöht erheblich die Flexibilität des Einsatzes des Instrumentariums.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:
- Fig. 1: zeigt perspektivisch den Zusammenbau aus einem Ankerelement und einem von diesem seitlich bogenförmig abstehenden Fadenabschnitt, der in den Ringkörper mündet,
- Fig. 2: eine entsprechende Darstellung, wobei hier zur Stabilisierung des Ringkörpers dieser um ein Ringelement gelegt ist,
- Fig. 3: den kompletten Zusammenbau eines Instrumentariums, wobei hier eine Ausführung gezeigt ist, bei der der Faden am Ankerelement in einer umfänglichen Nut befestigt ist, als Doppelstrang zu dem Ringkörper geführt ist und dieser Doppelstrang in einer seitlichen Führung eines Bügels aufgenommen ist, gleichzeitig ist auf das Ankerelement ein Eintreibwerkzeug aufgesetzt,
- Fig. 4: stark schematisiert eine Seitenansicht der Bauelemente von Fig. 1, wobei dargestellt ist, wie der Abschnitt des Fadens zwischen Ankerelement und Ringelement um ein zu fixierendes strangförmiges Gewebe legbar ist, und
- Fig. 5: das Ankerelement mit am Knochen fixiertem Gewebe.

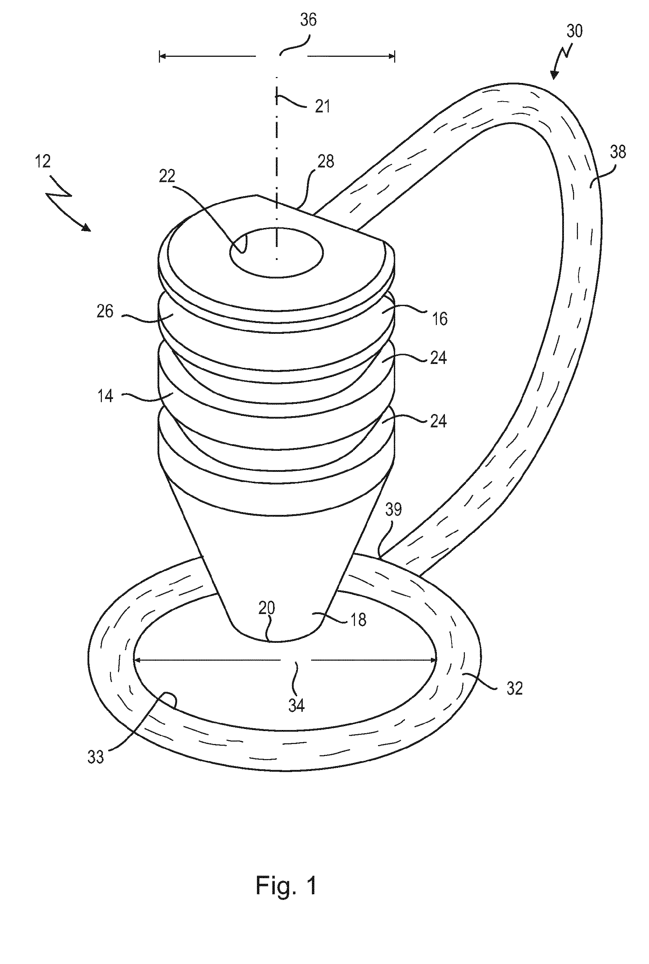

Ein in den Fig. 1 bis 5 dargestelltes Instrumentarium ist in seiner Gesamtheit mit der Bezugsziffer 10 bezeichnet.

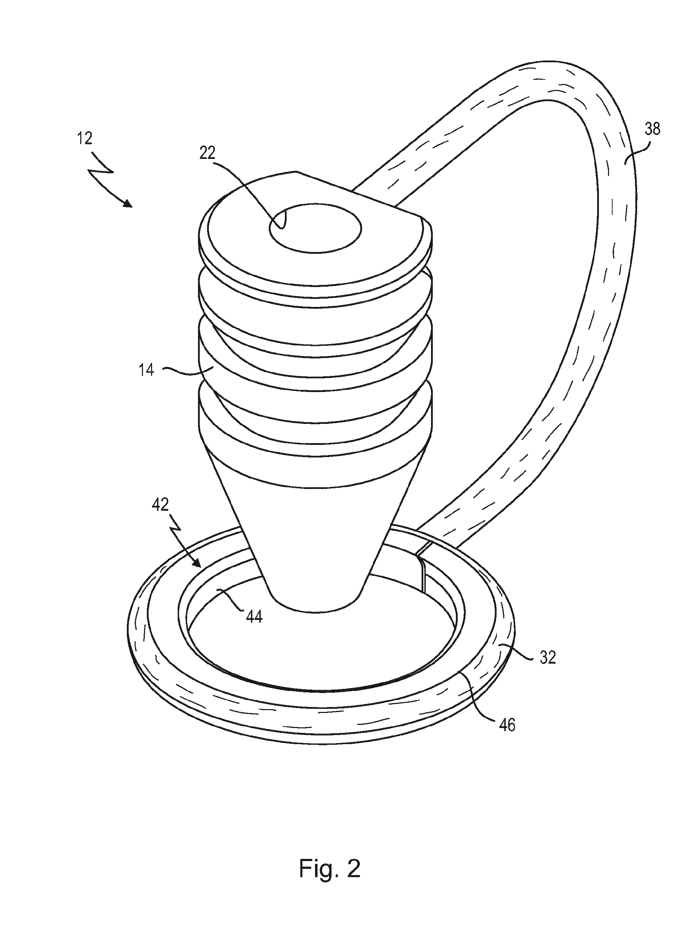

Das Instrumentarium 10 weist als eine wesentliche Baugruppe das in den Fig. 1 und 2 dargestellte Ankerelement 12 auf, das die Form eines Bolzens 14 aufweist.

Der Bolzen 14 weist einen massiven zylindrischen Körper 16 auf, der distalseitig in eine verjüngende Spitze 18 übergeht. Die Spitze ist als Kegelstumpf 20 ausgebildet.

Mittig durch das Ankerelement 12 hindurch längs dessen Längsachse 21 erstreckt sich eine durchgehende Bohrung 22. Diese Bohrung 22 kann am proximalen Endbereich auch mit einem Innengewinde versehen sein.

An der Außenseite des zylindrischen Körpers 16 sind verschiedene Einschnitte 24 vorgesehen, die dazu dienen, als Widerhaken zu wirken, nachdem das Ankerelement 12 in einen Knochen eingetrieben ist.

Der zylindrische Körper 16 weist in der Nähe seines proximalen Endes eine umlaufende Ringnut 26 auf. In diesem proximalen Endbereich weist der zylindrische Körper 16 eine Abflachung 28 auf.

Das Ankerelement 12 ist mit einem Faden 30 verbunden. Dieser Faden 30 weist an seinem von dem Ankerelement 12 entfernt liegenden Ende einen Schlaufenabschnitt 32 auf, der zu einem Ringkörper 33 geformt ist.

Dabei ist der lichte Innendurchmesser 34 des Ringkörpers 33 so gewählt, dass dieser etwas größer ist als der Außendurchmesser 36 des zylindrischen Körpers 16, so dass der zylindrische Körper 16, in der Darstellung von Fig. 1 von oben nach unten gerichtet, durch das Innere des Ringkörpers 33 hindurch bewegt werden kann.

Zwischen dem zylindrischen Körper 16 und dem Ringkörper 33 erstreckt sich ein seitlich ausgebogener Abschnitt 38 des Fadens.

In Fig. 1 ist dargestellt, dass der Ringkörper 33 als ein in sich geschlossener Ring aus dem Fadenmaterial besteht, der fest mit dem Abschnitt 38 verbunden ist.

Der Abschnitt 38 ist an seiner dem Ringkörper 33 gegenüberliegenden Ende am proximalen Ende des zylindrischen Körpers 16 befestigt.

Dies kann beispielsweise dadurch geschehen, dass dieses Ende in eine hier nicht näher gezeigte Öffnung in dem zylindrischen Körper 16 eingeführt und dort befestigt ist, was schon herstellerseitig durchgeführt werden kann.

Es ist aber auch möglich, dieses Ende des Fadens 30 dadurch am Ankerelement 12 zu befestigen, dass dieses in die Ringnut 26 eingelegt und im Bereich der Abflachung 28 verknotet wird. In dieser Ausgestaltung ist dann die Länge des Abschnittes 38 entsprechend variierbar.

Es ist aber auch möglich, den Faden 30 so auszubilden, dass er vom zylindrischen Körper 16 ausgehend als Doppelstrang ausgebildet ist, der sich dann zu dem Ringkörper 33 aufteilt. Dann müssen aber Vorkehrungen getroffen werden, um den Ringkörper 33 als Ring aufrecht zu erhalten. Das kann z.B. durch eine entsprechende plastische Verformbarkeit des Materials des Fadens bewerkstelligt werden. An der Abzweigstelle 39, also an der Stelle, an der der Doppelstrang den Ringkörper verlässt, kann ein entsprechender Clip oder eine Klebestelle gesetzt werden, um die Ringgeometrie des Ringkörpers 33 aufzuhalten. In diesem Fall ist dann der Ringkörper einsträngig ausgebildet.

In Fig. 2 ist eine Ausgestaltung dargestellt, bei der der Ringkörper 33 um ein Ringelement 42 gelegt ist.

Das Ringelement 42 besteht dabei aus einem metallischen steifen stabilen Ring 44, an dessen umfänglicher Außenseite eine Aussparung 46 vorgesehen ist, beispielsweise eine Nut, in die passend von der Außenseite her der Ringkörper 33 eingelegt bzw. eingedrückt ist.

Je nachdem, wie das herstellerseitig gewünscht ist, kann, wenn der Ringkörper 33 als durchgehender vorgefertigter Ring ausgebildet ist, oder der Ring 44 kann als geschlitzter Ring ausgebildet sein, so dass das Überstülpen oder Einlegen erleichtert ist. Weist das Material des Fadens 30 eine gewisse Elastizität auf, kann dieser Ringkörper 33 einfach über einen geschlossenen Ring übergestülpt werden. Durch die Ausgestaltung von Fig. 2 ist sichergestellt, dass der Ringkörper 33 auch bei biegeschlaffem Fadenmaterial seine Ringgeometrie beibehält, denn diese ist durch das Ringelement 42 vorgegeben.

Es ist auch möglich, schon herstellerseitig den Zusammenbau wie in Fig. 2 dargestellt vorzuproduzieren.

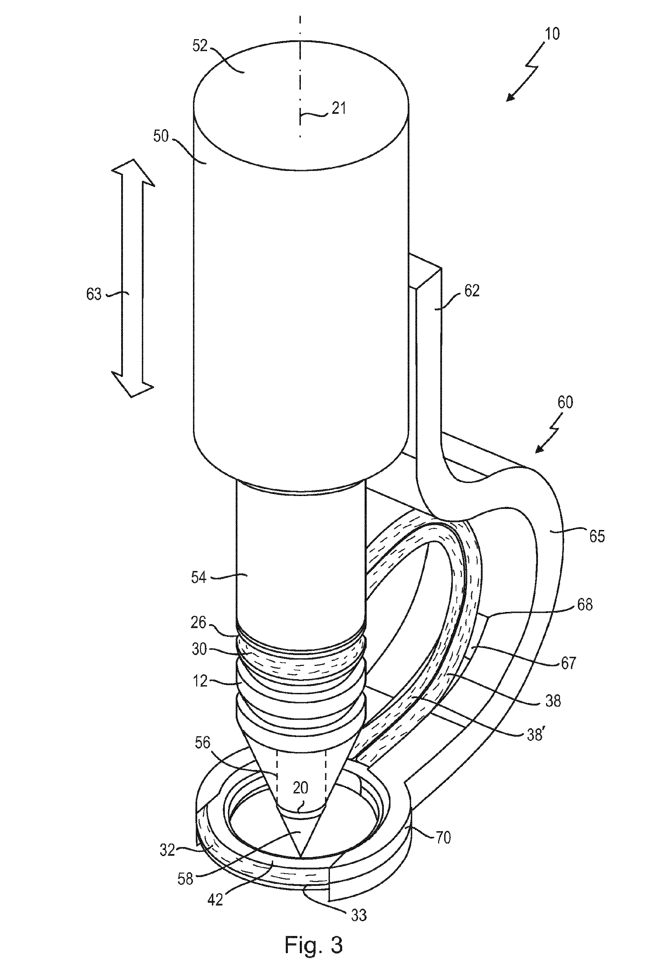

In Fig. 3 ist nun dargestellt, wie weitere Bauteile des Instrumentariums 10 ausgestaltet und zu handhaben sind.

Hier ist zunächst ein Eintreibwerkzeug 50 zu erkennen, das proximalseitig einen Schlagkopf 52 aufweist, der distalseitig in einen etwas dünneren Stab 54 übergeht.

Vom Stab 54 selbst springt distalseitig eine Stange 56 vor, die an ihrem äußeren Ende mit einer Spitze 58 versehen ist.

Der Außendurchmesser der Stange 52 und deren Länge ist so bemessen, dass diese durch die durchgehende Bohrung 22 von proximal nach distal hindurchgeschoben werden kann. Ist in der Bohrung 22 das zuvor erwähnte Innengewinde vorhanden, ist an der Außenseite der Stange 52 ein entsprechendes Außengewinde vorhanden, so dass durch die Schraubverbindung ein fester unverlierbarer Sitz des Ankerelements 12 am Eintreibwerkzeug 50 besteht. Nach dem Eintreiben wird das Eintreibwerkzeug 50 vom in den Knochen eingetriebenen Ankerelement 12 abgedreht. Dabei überragt die Spitze 58 den Kegelstumpf 20 des Ankerelementes 12 und ist so ausgebildet, dass sie den Kegelstumpf 20 zu einem spitzen Kegel ergänzt. Das Material der Stange 56 und der Spitze 58 ist aus strapazierfähigem metallischem Material, beispielsweise Medizinerstahl, hergestellt.

Die hier nicht näher bezeichnete Ringschulter in dem Übergang von dem Stab 54 zur durchmessergeringerem Stange 56 dient als Anlage auf dem ebenen proximalen Ende des zylindrischen Körpers 16 des Ankerelementes 12.

Das Eintreibwerkzeug 50 ist an einer seitlichen Haltevorrichtung 60 höhenverstellbar angebracht, wie das durch den Doppelpfeil 63 angedeutet ist.

Die Haltevorrichtung 60 weist dazu eine Schiene 62 auf, an der der Schlagkopf 52 höhenverstellbar geführt ist.

Die Schiene 62 setzt sich in einem seitlich ausbauchenden Bügel 65 fort, an dessen unterem Ende eine Klammer 70 angeordnet ist. Die Größe, die Öffnung und die Ausrichtung der Klammer 70 ist so ausgebildet, dass darin der in Fig. 2 dargestellte Zusammenbau von Ring 44 und Ringkörper 33 aus dem Fadenmaterial seitlich eingeclipst werden kann.

In Fig. 3 ist dargestellt, dass der Abschnitt des Fadens 30 zwischen dem Ringkörper 33 und dem Ankerelement 12 als Doppelstrang 38, 38' ausgebildet ist, der einmal um die Ringnut 26 geführt ist und auf Seiten der Abflachung verknotet ist.

Hier ist der Faden 30 als ein ursprünglich einsträngiger Faden einer bestimmten Länge auszubilden, er kann dann von außen in das Ringelement 42 eingelegt werden und die von diesem vorstehenden Stränge 38, 38' werden dann in einer inneren Ausnehmung 67 des Bügels 65 zum proximalen Ende des Ankerelementes 12 geführt und dort wie zuvor beschrieben befestigt.

In dem in Fig. 3 dargestellten Zustand ist das Instrumentarium 10 nun für einen Eingriff einsatzbereit.

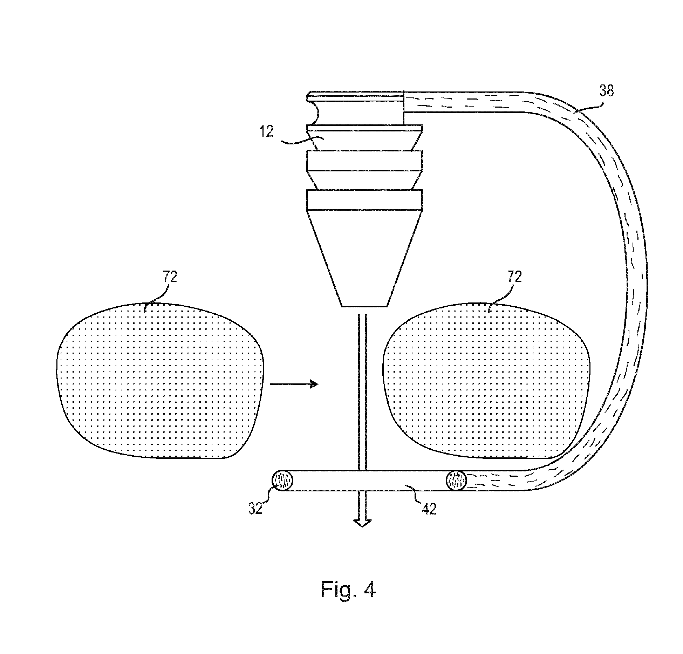

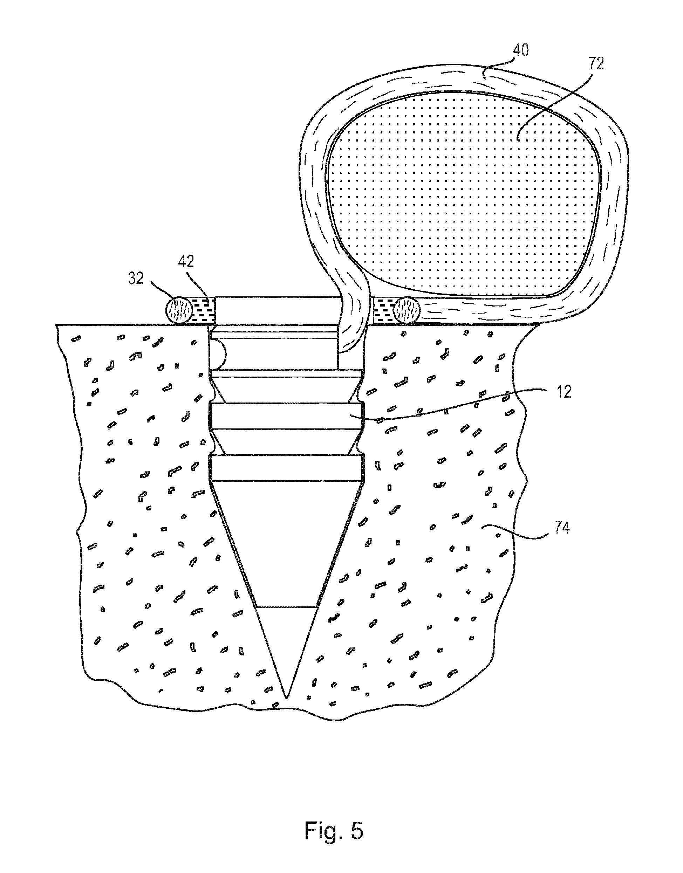

In Fig. 4 und 5 soll das stark schematisiert und der Übersichtlichkeit halber ohne das eigentliche Eintreibwerkzeug und die Halterung dargestellt werden.

Aufgrund der Höhenverstellbarkeit kann das Eintreibwerkzeug 50 samt dem daran angebrachten Ankerelement 52 soweit angehoben werden, dass ein zu befestigender Sehnenstrang 72 in den gebogenen Bereich bzw. Abschnitt 38 des Fadens 30 gebracht werden. Dabei kann das Instrumentarium 10 dann von einer Seite her an den Sehnenstrang 72 angelegt werden, in der Darstellung von Fig. 4 also von rechts nach links bewegt werden.

Im Endeffekt wird dann der Sehnenstrang 72 in diesen inneren Raum verbracht. Nunmehr kann das Ankerelement 12 bei dem in Fig. 3 dargestellten Bauzustand von oben durch das Ringelement 42 hindurchgetrieben werden. Dazu wird die Unterseite des Ringelementes 42 auf eine Knochenoberfläche gelegt und zwar an einer Stelle, die sowohl günstig für die Fixierung des Sehnenstranges 72 ist als auch günstig für das Eintreiben des Ankerelementes 12. Nach dem Eintreiben, wie das aus Fig. 5 ersichtlich ist, ist das Ankerelement 12 vollständig in den Knochen 74 eingedrungen.

Der Abschnitt 38 hat sich nunmehr um die Außenseite des Sehnenstranges 72 gelegt, und bildet somit eine Schlaufe 40, durch die der Sehnenstrang 72 an den Knochen 74 herangelegt und damit fixiert wird.

Aus Fig. 5 ist ersichtlich, dass das Ringelement 42 auf der Außenseite des Knochens 74 verbleibt.

Die notwendige Spannung auf die Schlaufe 40 kann dadurch erzielt warden, dass das Ankerelement 12 aus der in Fig. 5 dargestellten Position noch etwas weiter in den Knochen 74 eingetrieben wird.

Hier ist ersichtlich, dass die umfängliche Länge der Schlaufe 40 in etwa der Länge des Abschnittes 38, wie er in Fig. 4 dargestellt ist, entspricht. Man kann daher vor dem Eingriff, wenn man den Außenumfang des Sehnenstranges 72 weiß, die Länge entsprechend auswählen.

Wird die zuvor erwähnte Ausgestaltung herangezogen, bei der der Faden als Doppelstrang im Bereich der Verbindungsstelle zwischen dem Ringkörper 33 und dem Ankerelement 12 ausgebildet ist und ist kein solches Ringelement 42 vorhanden, kann auch dieser Fadenbereich, also über die Länge des Umfanges des Ringkörpers 33 mit zur Ausbildung der Schlaufe 40 herangezogen werden, was dann bei der entsprechenden Längenausbildung des Abschnittes 38 berücksichtigt wird. D.h., es ist möglich, wenn der Ringkörper 33 ausreichend stabil hergestellt werden kann, dieses Instrumentarium auch ohne das Ringelement 42 zu verwenden.

Es ist auch möglich, den im Ringelement 42 aufgenommenen Fadenbereich zur Befestigung heranzuziehen, indem dieser Fadenbereich aus dem Ringelement 12 herausgenommen wird. Dann ist die halbe Länge des Umfanges des Ringelements für die Ausbildung der Schlaufe 40 hinzuzurechnen.

## Patentansprüche

1. Instrumentarium zum knotenfreien Fixieren von Gewebe (72) an einem Knochen (74), mit einem bolzenförmigen, eine Längsachse (21) aufweisenden Ankerelement (12), das mit einem Faden (30) verbunden ist, der einen endseitigen Schlaufenabschnitt (32) aufweist,
**dadurch gekennzeichnet, dass** der Schlaufenabschnitt (32) zu einem Ringkörper (33) geformt ist, dessen lichter Durchmesser (34) so gewählt ist, dass das Ankerelement (12) durch den Ringkörper (33) hindurch bewegbar ist, und dass eine Länge des Abschnittes (38) des Fadens (30) zwischen dem Ankerelement (12) und dem Ringkörper (33) derart aufwählbar ist, dass, nach dem Durchbewegen des Ankerelementes (12) durch den Ringkörper (33), eine Schlaufe (40) ausbildbar ist, die umfänglich eng um ein zu fixierendes strangförmiges Gewebestück (72) liegt.

2. Instrumentarium nach Anspruch 1, **dadurch gekennzeichnet, dass** der Schlaufenabschnitt (32) um einen Ringelement (42) geführt ist.

3. Instrumentarium nach Anspruch 2, **dadurch gekennzeichnet, dass** das Ringelement (42) als Ring (44) ausgebildet ist, an dessen umfänglicher Außenfläche eine Aussparung (46) vorhanden ist, in die der Schlaufenabschnitt (32) einlegbar ist.

4. Instrumentarium nach Anspruch 3, **dadurch gekennzeichnet, dass** die Aussparung (46) derart ausgebildet ist, dass der Fadenabschnitt (32) abrutschsicher anliegt.

5. Instrumentarium nach einem der Ansprüche 1 bis 4, **dadurch gekennzeichnet, dass** das Ankerelement (12) einen proximale Anbringstelle aufweist, an der der Faden (30) anbringbar ist.

6. Instrumentarium nach Anspruch 5, **dadurch gekennzeichnet, dass** die Anbringstelle eine umfängliche Nut (26) an der Außenseite des Ankerkörpers (12) aufweist, in die das ankerseitige Ende des Fadens (30) einlegbar ist.

7. Instrumentarium nach einem der Ansprüche 1 bis 6, **dadurch gekennzeichnet, dass** am proximalen Ende des Ankerelements (12) ein Eingriffsmerkmal für ein Eintreibwerkzeug (50) vorgesehen ist.

8. Instrumentarium nach einem der Ansprüche 1 bis 7, **dadurch gekennzeichnet, dass** das Ankerelement als massiver zylindrischer Körper (16) ausgebildet ist, dessen distales Ende als kegelartige Spitze (18) ausgebildet ist.

9. Instrumentarium nach Anspruch 7 oder 8, **dadurch gekennzeichnet, dass** das Eingriffsmerkmal eine sich in Richtung der Längsachse (21) erstreckende Bohrung (22) im Körper (16) ausgebildet ist.

10. Instrumentarium nach Anspruch 9, **dadurch gekennzeichnet, dass** die Bohrung (22) als durch den Körper (16) vollständig hindurchgehende Bohrung (22) ausgebildet ist, dass die kegelartige Spitze (18) als Kegelstumpf ausgebildet ist, und dass ein in die Bohrung (22) eingeführtes Eintreibwerkzeug (50) eine Spitze (58) aufweist, die den Kegelstumpf (20) zu einem spitzen Kegel ergänzt.

11. Instrumentarium nach Anspruch 10, **dadurch gekennzeichnet, dass** eine Haltevorrichtung (60) vorgesehen ist, an der ein mit dem Ankerelement (12) verbundenes Eintreibwerkzeug (50) verbindbar ist, und die distalseitig eine Klammer (70) aufweist, in die das Ringelement (42) samt dem darin eingelegten Fadenabschnitt seitlich einclipsbar ist.

12. Instrumentarium nach Anspruch 11, **dadurch gekennzeichnet, dass** die Haltevorrichtung (60) eine seitlich zur Längsachse (21) verlaufende Führung (68) für den Fadenabschnitt (38) zwischen Ringkörper (38) und Ankerelement (12) aufweist.

13. Instrumentarium nach Anspruch 12, **dadurch gekennzeichnet, dass** die Führung (68) als seitlich ausbauchender, hochstehender Bügel (65) ausgebildet ist, an dessen dem Fadenabschnitt (38, 38') zugewandten Seite eine Ausnehmung (67) zur Aufnahme des Fadenabschnittes (38, 38') aufweist.

14. Instrumentarium nach Anspruch 13, **dadurch gekennzeichnet, dass** das Eintreibwerkzeug (50) höhenverstellbar am proximalen Ende des Bügels (65) angebracht ist.

15. Instrumentarium nach einem der Ansprüche 11 bis 14, **dadurch gekennzeichnet, dass** die Klammer (70) am proximalen Ende des Bügels (65) angeordnet ist, und den Ringkörper (33) des Fadens (30) in einer solchen Ausrichtung hält, dass der Ringkörper (33) an eine Außenseite des Knochens (74) flächig anlegbar ist.

16. Instrumentarium nach Anspruch 15, **dadurch gekennzeichnet, dass** der Ringkörper (33) in einer Ausrichtung in der Klammer (70) gehalten ist, bei der die Längsachse (21) des Ankerelementes (12) mittig senkrecht auf der Ringebene steht.
